# EUROPEAN PATENT APPLICATION

(11) **EP 1 703 697 A1**
(43) Date of publication of application: **20.09.2006**
(21) Application number: 06251358.5
(22) Date of filing: 14.03.2006
(51) Int. Cl.: H04L 29/06, A61B 5/00, H04B 5/00, H04B 1/00, H04L 29/08

(54) **Wireless acquisition and monitoring system**

(30) Priority: 14.03.2005 US 661803 P; 09.03.2006 US 373801
(71) Applicant: Alfred E. Mann Foundation for Scientific Research, Santa Clarita, CA 91380-9005 (US)
(72) Inventor: Schulman, Joseph H., Santa Clarita, CA 91387 (US); Mobley, Jon Phil, Canyon Country, CA 91387 (US)
(74) Representative: Carter, Stephen John

(57) **Abstract**

A sensor device (12) is positioned to monitor a desired parameter and transmit a corresponding signal representative of such parameter, to a remote apparatus (14) via a wireless communication link. The remote apparatus (14) converts the received signal to a form appropriate for display on a display device (16), such as an oscilloscope. The desired parameter monitoring and display are in real time. A plurality of sensor devices (12) is contemplated with each sensor device (12) having a unique identification code. When the remote apparatus issues command and control data to a selected sensor device, it does so in association with the identification code of such selected sensor device.

## Description

### Field of the Invention

An aspect of the instant invention generally relates to a wireless monitor probe and more particularly to a system having a sensor device for monitoring a desired parameter and a remote apparatus in wireless communication with the remote device to command and control the device and convert data received from the sensor device related to the desired parameter into a form for display on a display device.

### Summary of Invention

In one aspect the invention provides a remote monitoring system as set forth in claim 1.

### Description of the Drawings

FIG. 1 is simplified block diagram of system of an embodiment of the present invention comprising a sensor device, a remote apparatus, and a display.

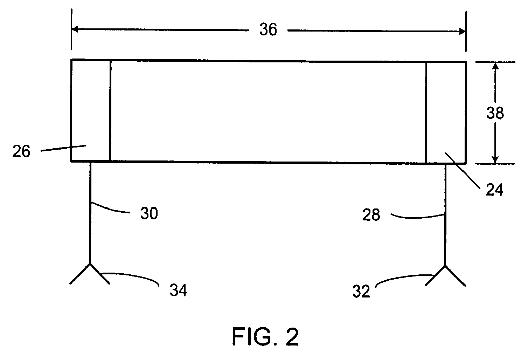

FIG. 2 is a front view of the sensor device shown in FIG. 1.
FIG. 3 is a block diagram of the sensor device and remote apparatus of the system shown in FIG. 1.

### Detailed Description

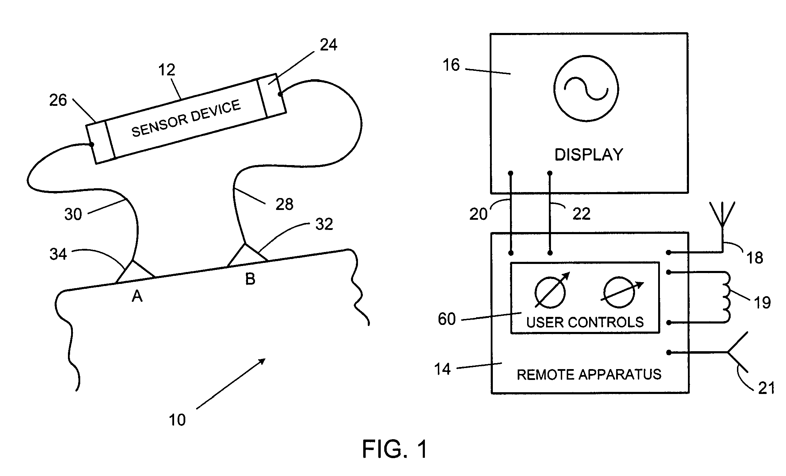

Referring to FIG. 1 there is shown an illustration of system 10 for providing remote monitoring of a desired parameter. More specifically, the system 10 includes a sensor device 12, a remote apparatus 14, and a display 16. The remote apparatus is positioned at a location remote from sensor device 12, where the sensor device is to monitor a desired parameter. The sensor device 12 is an extra corporeal device, and although it may be adapted for implant, the sensor device is typically not for use for implant in an mammalian body, but rather for use in parameter monitoring applications outside of a body, or at best, on the outer surface of a body. The sensor device 12 may be configured to be adapted for implant in a mammalian body by one skilled in the art. The remote apparatus 14 provides command, interrogation requests and status information to the sensor device 12 by means of a wireless communication link. In operation, the wireless communication link comprises a modulated magnetic signal, an ultrasonic signal, or a radio frequency (RF) signal. The signal strength requirements are sufficient to provide a wireless link over at least a 10 meter range.

In an embodiment of the invention, wireless communication between the remote apparatus 14 and the sensor device 12 is accomplished in part by the use of antenna 18. Wireless communication can be implemented by the use of telemetry signals or signals in an RF range. To be discussed below, the RF signal may be detected and demodulated by receiver circuitry contained within sensor device 12.

In an alternate embodiment, an alternating magnetic field may be amplitude modulated and transmitted by remote apparatus 14 by means of transmission/receiver coil 19. The receiver circuitry is configured to demodulate the magnetic field signal in a manner to produce data and command information consistent with further processing by the sensor device 12.

In yet another alternate embodiment, an ultrasonic signal can be used to deliver data and command signals to the sensor device 12. In this embodiment, an ultrasonic transmitter 21 coupled to remote apparatus 14 transmits data and command information to the sensor device 12. The sensor device receiver circuitry is coupled to an ultrasonic transducer whose output is demodulated and processed in a manner similar to that in reference to the RF signal. The remote apparatus is coupled to display 16 by means of signal wires 20 and 22.

The display 16 may be any one of a number of display devices, such as for example but not limited to, a monitor screen, an oscilloscope, digital read out monitor or television screen. The display of choice is dependant, in part, on the nature of the signal to be monitored by the sensor device 12. For example, if the desired parameter is a rapidly time variable signal, then a cathode ray tube (CRT) oscilloscope is a suitable display. On the other hand, if a non-variant or slowly varying parameter is monitored, then a direct current type meter may be suitable. The sensor device 12 may comprise a microstimulator device similar to that described in U.S. Patent 6,185,452 incorporated herein in its entirety by reference. Additionally, the apparatus and method utilized in the transfer and communication of data, status, power and command information and signals between the sensor device 12 and remote apparatus 14 is similar to that described in the '452 patent.

The sensor device 12 includes two electrodes 24 and 26 capable of detecting electrical signals appearing across the electrodes. In those instances where it is desirable to monitor signals not accessible directly by immediate contact with electrodes 24 and 26, a pair of leads 28 and 30 may be attached to electrodes 24 and 26 respectively. The distal ends of leads 28 and 30 may include attachment devices 32 and 34 respectively. The attachment devices 32 and 34 may, for example, be in the form of "alligator clips" and the like appropriate to attach the sensor device 12, for example, to discrete electrical components in an electrical circuit to be probed at desired locations. In other applications, the attachment device may be any suitable probe capable of making direct contact with the area to be probed. Furthermore, the leads 28 and 29 may be configured to transmit processed detected electrical signals appearing across the electrodes 24 and 26.

The sensor device 12 may be contained in a hermetically sealed housing, as for example, as described in the '452 patent or in one of many other housings, such as plastic or metal, where hermeticity may not be a requisite. Furthermore, although an embodiment of the invention includes a sensor device 12 having a lateral dimension of no greater than about 6mm and an axial dimension of no greater than about 60mm, alternate embodiments of the sensor device 12 may be of greater dimension, both in the lateral and axial sense. Moreover, a power source for the sensor device 12 may be made to have a power capacity of at least one-microwatt hour. A lithium iodide (LI-1) battery suitable for such use has an energy density of about 240 mw-hr/cm³. The battery typically has a voltage of about 3.6 volts, which is adequate for operating CMOS circuits. Where greater energy capacity is desirable standard hearing aid or photo camera batteries, as well as AAA to D batteries including rechargeable batteries may also be suitable. The larger capacity batteries may be adequate to support operation of a sensor device 12 for a much longer period of time than the lower capacity one micro watt/hour LI-I battery.

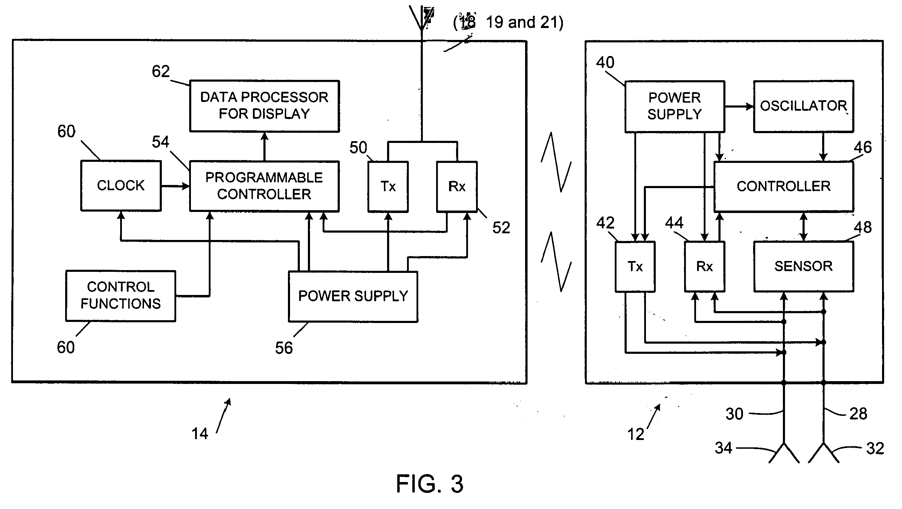

FIG. 3 shows a system comprising a sensor device 12 and the remote apparatus 14 in detail to sense a desired parameter. The sensor device 12 comprises a (1) power supply 40 as previously discussed, (2) a signal transmitter [Tx] 42 (when transmitting sense parameter values by means of leads 28 and 30), (3) a signal receiver [Rx] 44 for receiving command signals from remote apparatus 14, (4) a controller or state machine 46 responsive to receive signals from the apparatus 14 for controlling the time for monitoring the desired parameter and transmitting signals related to the desired parameter back to the apparatus 14, and (5) a sensor 48 coupled to leads 28 and 30 that is configured to convert signals appearing across leads 28 and 30 in a form appropriate for processing by controller 46. In those instances when environmental conditions, such as temperature are to be monitored, the sensor 48 may be a temperature monitor such as a thermistor whose output provides a signal related to the monitored temperature provided to the controller 46 for processing and transmission to remote apparatus 14.

The remote apparatus 14 comprises (1) a signal transmitter [Tx] 50 for transmitting command signals to sensor device 12, (2) a signal receiver [Rx] 52 for receiving status and desired parameter information from sensor device 12, (3) a programmable controller 54 responsive to received status signals for providing command signals for transmission by the transmitter 50, (4) a power supply 56 to provide power for operation of the remote apparatus 14, (5) a clock 58 to provide an accurate signal frequency source for data processing, clocking, and setting the rate at which command instructions are executed, and (6) the control functions block 60 which provides a user control panel for providing system operational commands by the user, such as "commence parameter monitoring", and (7) a data processor for display 62 configured to provide monitored desired parameter information in a form appropriate for the particular display device to be used. As previously discussed, if the desired parameter is a time varying signal, then the data processor 62 would provide a signal appropriate for a display device such as an oscilloscope capable of displaying a time varying signal.

The sensor device 12 is remotely controlled to sense parameter information in a manner as described in U.S. Patent 6,164,284 assigned to the same assignee as this application and incorporated herein by reference in its entirety. Although the foregoing discussion involves only one remote sensor 12, it is to be understood that multiple sensor devices 12 may be distributed in a zone in which communication with the remote apparatus 14, and an individual sensor device 12, is achievable. In order to distinguish each sensor device 12 over the communication channel, each sensor device 12 is implemented with an identification code specified either in controller 46 or a conventional storage location, i.e., address storage circuitry, easily implemented by one skilled in the art in a manner for communicating between the remote apparatus 16 and a plurality of remote devices 12. Such technique is described in U.S. Patent 6,472,991 assigned to the same assignee as this application, and incorporated herein by reference in its entirety.

Accordingly, the remote apparatus 14 through control functions 60 provides capability to selectively monitor the outputs of such selected sensor devices. By transmitting an interrogation command, including the identification code, the remote apparatus can access directly the desired sensor device for desired parameter monitoring and observation. The programmable controller 54 contains circuitry for sequentially accessing a plurality of sensor devices 12 in a rapid multiplexing mode so as to provide real time monitoring of multiple parameters for multiple display on a display device typically an oscilloscope. Such circuitry causes the remote apparatus to issue monitor commands with associated identification number in rapid succession so as to provide a capability of simultaneous multi-trace multiple desired parameter display on the display device. A typical but by no means exclusive application for an embodiment of the present invention contemplates the use of one or more sensor devices used as temporary or permanent probes in an electrical circuit to monitor various electrical signals of interest. In this manner circuit response to various inputs and conditions can be monitored and measured in real time. In this manner, the necessity of having electrical conductors extending distances, whether short or long, between sensors and monitoring equipment with all the inherent problems normally accompanying such an arrangement is obviated.

With all the forgoing described embodiments of the invention, it is evident that the parameter monitoring capability, so described, obviates a necessity for multiply conductor wires and leads between sensors, remote apparatus (controllers), and display devices. Accordingly, undesirable interference signals, live noise, ground loops, stray electric and magnetic fields are significantly, if not totally, eliminated as to their effect on desired parameter value integrity. Moreover, access to monitor points is facilitated due to the absence of monitor leads and wires.

## Claims

1. A remote monitoring system comprising:
a sensor device adapted to monitor a desired parameter;
a remote apparatus adapted for controlling, via a wireless communication link, the sensor device, the remote apparatus being positioned at a location remote from the sensor device, said remote apparatus adapted for transferring data relating to the desired parameter and/or commands between the sensor device and the remote apparatus via said wireless communication link; and
a display coupled to the remote apparatus adapted to provide a display corresponding to the desired parameter.

2. The system of claim 1, wherein the sensor device, the remote apparatus and the display are configured to monitor, transfer data relating to and display, respectively, the desired parameter in real time.

3. The system of claim 1, wherein the sensor device is contained within a housing comprising an axial dimension of no greater than about 60 mm and a lateral dimension no greater than about 6 mm.

4. The system of claim 1, wherein the sensor device comprises a hermetically sealed housing.

5. The system of claim 1, wherein the sensor device comprises at least two electrodes, the electrodes adapted to receive command data from the remote apparatus and to transmit monitored desired parameter data to the remote apparatus.

6. The system of claim 5, wherein each electrode comprises an attachment device adapted to secure the electrodes at the location where the desired parameter is monitored.

7. The system of claim 6, wherein the attachment device is adapted to be attached at a desired location in an electrical circuit and wherein the desired parameter comprises a parameter of the electrical circuit.

8. The system of claim 1, wherein the display comprises an oscilloscope.

9. The system of claim 1, wherein the wireless communication link comprises a telemetry link.

10. The system of claim 1, wherein the wireless communication link comprises a radio frequency transmission link.

11. The system of claim 1, wherein the sensor device is an implantable medical device, said medical device being contained in a hermetically sealed housing.

12. The system of claim 11, wherein the desired parameter comprises electrical signals developed by a mammalian body.

13. The system of claim 1, wherein the sensor device comprises software configured to program the sensor device to perform selected functions.

14. The system of claim 1, wherein the remote apparatus comprises an antenna adapted to provide a wireless communication link between the remote apparatus and the sensor device.

15. The system of claim 1, wherein the sensor device comprises software configured to program the sensor device to perform selected functions.

16. The system of claim 1, wherein the remote apparatus comprises an antenna adapted to provide a wireless communication link between the remote apparatus and the sensor device.

17. The system of claim 1, wherein the sensor device comprises a plurality of sensor devices, each sensor device of the plurality having a unique identification code associated therewith, a selected sensor device responding to an interrogation or command signal when such signals include the identification code of such selected sensor device.

18. The system of claim 17, wherein the remote apparatus comprises means for providing the unique identification code in association with interrogation or command signals for a selected sensor device to which such interrogation or command signals are to be transmitted.

19. The system of claim 1, wherein said sensor device comprises a power source comprising a battery having a capacity of at least one-microwatt-hour.
